# FASCICULE DE BREVET EUROPEEN

(11) **EP 0 683 681 B1**
(45) Date de publication et mention de la délivrance du brevet: **31.07.2002**
(21) Numéro de dépôt: 95903391.1
(22) Date de dépôt: 09.12.1994
(51) Int. Cl.: A61L 27/00, A61K 35/56

(54) **IMPLANT INJECTABLE POUR LA CORRECTION DES RIDES ET DES DEPRESSIONS DERMIQUES**
INJIZIERBARES IMPLANTAT ZUR BEHANDLUNGEN VON FALTEN UND HAUTDEPRESSIONEN
INJECTABLE IMPLANT FOR CORRECTING WRINKLES AND SKIN HOLLOWS

(30) Priorité: 10.12.1993 FR 9314964
(43) Date de publication de la demande: 29.11.1995
(73) Titulaire: FORTUNE BASE MANAGEMENT, LTD., Central Hong Kong (HK)
(72) Inventeur: Camprasse, Georges, 77480 Villenauxe-la-Petite (FR); Camprasse, Serge, 77500 Chelles (FR)
(74) Mandataire: Thinat, Michel
(86) Numéro de dépôt international: FR9401447
(87) Numéro de publication internationale: WO9515774

(56) Documents cités:
- FR-A- 2 502 007
- FR-A- 2 590 168
- FR-A- 2 595 247

## Description

La présente invention concerne une substance extraite du fluide extrapalléal des mollusques bivalves gastropodes, céphalopodes, traitée de façon à en modifier la structure moléculaire afin de la rendre biocompatible, destinée à la réparation du derme et de l'hypoderme.

Jusqu'à maintenant le seul matériau implantable par injection le plus largement utilisé pour corriger les dépressions dermiques est le collagène d'origine bovine. On a aussi utilisé les silicones, le Gortex, le Fibrel, le collagène et la graisse autologues. Toutefois, ces substances de par leur composition physico-chimique n'ont aucune action durable sur les différents composants du derme et de l'hypoderme. De plus, elles ont chacune leurs inconvénients qui demandent, lors de leur utilisation, des précautions destinées, pour certaines, à éviter des réactions d'hypersensibilité immédiates ou retardées.

En ce qui concerne le collagène d'origine bovine, on doit tenir compte d'une antigénicité dûe à l'origine d'extraction de ce biomatériau. En effet, plus de 8% de la population qui n'a jamais été en contact avec le collagène d'origine bovine, présente des anticorps anticollagène bovin.

Il fallait donc trouver, et c'est ce qui fait l'objet de la présente invention, une substance qui tout en permettant de corriger une dépression ou de restaurer une région dermique endommagée, aurait une action sur les différents composants structuraux de l'architecture hypodermique, sans les effets immunogènes cités plus haut.

Le produit selon l'invention se présente sous la forme d'un liquide incolore extrait par aspiration de la cavité extrapalléale des mollusques bivalves gastropodes, céphalopodes, tels les Ormaux, les Pinctada, le Nautile, le Buccin, le Calamar, le Bénitier.

Le produit selon l'invention est au préalable débarrassé de l'eau de mer, de préférence par évaporation sous vide à 40°C et est recueilli sous forme de gel.

Le produit selon l'invention est lavé, décontaminé, rincé abondamment et mis en solution dans de l'eau déminéralisée. Il subit un traitement à l'acide acétique afin d'éliminer les résidus d'ions de calcium contenus dans l'eau de mer.

Le produit ainsi obtenu est centrifugé, filtré et concentré par évaporation sous vide à 40%. Ce concentrat est mélangé à un éluant qui est de l'eau déminéralisée à pH 6.

Le produit ainsi obtenu est traité à l'étuve à 120° sous une pression de 1 bar.

On procède alors au dessalage par chromatographie, par perméation sur gel. Les composants organiques solubles débarrassés de leurs sels, sont concentrés sous vide et lyophilisés.

Le produit selon l'invention subit un traitement enzymatique destiné à supprimer les liaisons télopeptidiques responsables de l'antigénicité. Un tel traitement enzymatique préférentiel consiste à traiter le produit avec une hydrolase acide.

Il est dispersé dans du sérum physiologique tamponné au phosphate et se présente sous la forme d'un gel, et subit trois phases de stérilisation destinées à le rendre prêt à l'emploi. Les trois phases de stérilisation consistent de préférence d'un autoclavage à 121°C pendant 20 minutes suivi d'un traitement à la vapeur sèche (100°C) pendant 1 heure et enfin d'un traitement par rayonnement ionisant β ou γ.

La structure générale du produit selon l'invention peut être modifiée par création ou transposition de liaisons chimiques destinées à modifier son comportement et ses propriétés, par exemple physiques telles que la viscosité et le temps d'assimilation comme cela est connu de l'homme de l'art, sans que ces procédés soient limitatifs. Il peut aussi subir des polymérisations avec des composés protéiniques ou non protéiniques.

Afin de favoriser une injection indolore du produit selon l'invention, on peut y ajouter des anesthésiques locaux.

Le produit selon l'invention est présenté dans des seringues stériles préremplies, prêtes à l'emploi et contenant des concentrations différentes selon l'utilisation.

Les exemples suivants illustrent l'utilisation du produit selon l'invention :
- après désinfection de la peau, 80 mg du produit selon l'invention sont injectés dans le pli abdominal de 10 brebis jusqu'à gonflement et disparition de la dépression.
- dans un second cas,l'utilisation du produit selon l'invention, il a été réalisé une dermabrasion profonde de la région abdominale de 4 brebis avec altération du derme profond, le produit selon l'invention a été implanté par injections traçantes de façon à corriger la dépression.

Dans tous les cas, il n'a pas été noté de réactions inflammatoires locales, ni de réactions d'immunogénicité objectivées par des réponses cellulaires ou humorales. De plus on note une persistance de la correction des dépressions au-delà de 24 mois.

Ces observations démontrent de manière irréfutable que le produit selon l'invention corrige non seulement les dépressions, et les pertes de substances dermiques, mais encore, comme l'ont montré les études histologiques, stimule la synthèse et la régulation du collagène receveur avec régénération de l'élastine.

Il appartiendra à l'homme du métier de mettre en oeuvre le produit selon l'invention chaque fois qu'il sera nécessaire de corriger les dépressions dermiques, les rides, ou d'induire la synthèse du collagène humain.

## Revendications

1. Implant injectable, **caractérisé en ce qu'**il est obtenu à partir du fluide extrapalléal des mollusques bivalves, gastropodes, céphalopodes.

2. Implant injectable selon la revendication 1, **caractérisé en ce que** le fluide extrapalléal est celui des Pinctada, du Tridacne, des Ormaux, des Conques, du Buccin, du Nautine, du Calamar, du Bénitier.

3. Implant injectable selon les revendications 1 et 2, **caractérisé en ce qu'**il subit une décontamination, suivie d'un traitement décalcifiant à l'acide acétique, qu'il est traité à l'étuve sous une pression de un bar à 120°, qu'il subit un dessalage par chromatographie, par perméation sur gel, un traitement enzymatique d'antigénicité, qu'il est lyophilisé et dispersé dans du sérum physiologique tamponné sous forme de gel.

4. Implant injectable selon les revendications 1, 2 et 3, **caractérisé en ce qu'**il peut être associé à des substances protéiniques ou non protéiniques.

5. Utilisation du produit selon les revendications 1, 2, 3 et 4 pour le traitement et la correction des rides et des dépressions dermiques.

## Patentansprüche

1. Injizierbares Implantat, **dadurch gekennzeichnet, dass** es aus der Flüssigkeit außerhalb der Kiemenhöhlen zweischaliger Weichtiere, Bauchfüßler und Kopffüßler gewonnen wird.

2. Injizierbares Implantat gemäß Anspruch 1, **dadurch gekennzeichnet, dass** die Flüssigkeit außerhalb der Kiemenhöhlen die der Pinktada, der Riesenmuschel, der Seeohren, der Trompetenschnecke, der Wellhornschnecke, des Nautilus, des Tintenfischs, der Mördermuschel ist.

3. Injizierbares Implantat gemäß Anspruch 1 und 2, **dadurch gekennzeichnet, dass** es eine Dekontaminierung erfährt, gefolgt von einer entkalkenden Behandlung durch Essigsäure, dass es im Trockenofen mit einem Druck von einem Bar bei 220° behandelt wird, dass es eine Entsalzung durch Chromatographie, durch Permeation auf Gel und eine enzymatische Antigenbehandlung erfährt, dass es lyophilisiert und in einem physiologischen gepufferten Serum in Gelform dispergiert wird.

4. Injizierbares Implantat gemäß Anspruch 1, 2 und 3, **dadurch gekennzeichnet, dass** es eiweißähnlichen oder nicht eiweißähnlichen Substanzen zugeordnet werden kann.

5. Verwendung des Produkts gemäß Anspruch 1, 2, 3 oder 4 zur Behebung und Behandlung von Falten und Hautdepressionen.

## Claims

1. Injectable implant wherein it is obtained from the extrapalleal fluid of bivalve molluscs, gastropods and cephalopods.

2. Injectable implant according to claim 1 wherein the extrapalleal fluid is that of pinctada, tridacne, earshells, conchs, whelks, nautilus, squid.

3. Injectable implant according to claims 1 and 2 wherein it undergoes decontamination followed by decalcifying treatment with acetic acid, and wherein it is processed in the oven under pressure of 1 bar at 120°, undergoes desalination by chromatography, gel permeation, antigenic enzyme processing and is freeze-dried and dispersed in buffered physiological serum in the form of a gel.

4. Injectable implant according to claims 1, 2 and 3 wherein it can be combined with protein or non-protein substances.

5. Use of the product according to claims 1, 2, 3, and 4 for the treatment and correction of wrinkles and skin hollows.
